# EUROPÄISCHE PATENTSCHRIFT

(11) **EP 1 333 819 B1**
(45) Veröffentlichungstag und Bekanntmachung des Hinweises auf die Patenterteilung: **19.09.2007**
(21) Anmeldenummer: 01992567.6
(22) Anmeldetag: 24.10.2001
(51) Int. Cl.: A61K 9/72, A61K 31/5386

(54) **INHALATIVE LÖSUNGSFORMULIERUNG MIT EINEM TIOTROPIUMSALZ**
INHALATIVE SOLUTION FORMULATION CONTAINING A TIOTROPIUM SALT
FORMULATION D'UNE SOLUTION D'INHALATION CONTENANT UN SEL DE TIOTROPIUM

(30) Priorität: 31.10.2000 DE 10054036
(43) Veröffentlichungstag der Anmeldung: 13.08.2003
(60) Teilanmeldung: 07111499.5
(73) Patentinhaber: Boehringer Ingelheim Pharma GmbH & Co. KG, 55216 Ingelheim am Rhein (DE)
(72) Erfinder: NIKLAUS-HUMKE, Barbara, 55130 Mainz (DE); SCHMELZER, Christel, 55218 Ingelheim am Rhein (DE); BARTH, Petra, 55131 Mainz (DE); DRECHSEL, Karin, 55218 Ingelheim (DE)
(86) Internationale Anmeldenummer: PCT/EP2001/012292
(87) Internationale Veröffentlichungsnummer: WO 2002/036104

(56) Entgegenhaltungen:
- WO-A-00/23037
- WO-A-98/27959

## Beschreibung

Die vorliegende Erfindung betrifft eine treibgasfreie Inhalationsformulierung von einem in Wasser oder in einem Gemisch aus Wasser und Ethanol gelöstem pharmazeutisch akzeptablen Salz von Tiotropium und daraus resultierenden treibgasfreien inhalierbaren Aerosolen. Die erfindungsgemäße Formulierung eignet sich besonders zur inhalativen Applikation des Wirkstoffs, insbesondere in den Indikationen Asthma und COPD.

Tiotropium, chemisch (1α,2β,4β,5α,7β)-7-[(hydroxydi-2-thienylacetyl)oxy]-9,9-dimethyl-3-oxa-9-azoniatricyclo[3.3.1.0]nonane, ist als Tiotropiumbromid aus der Europäischen Patentanmeldung EP 418 716 A1 bekannt. Das Bromid-Salz des Tiotropiums weist die folgende chemische Struktur auf:

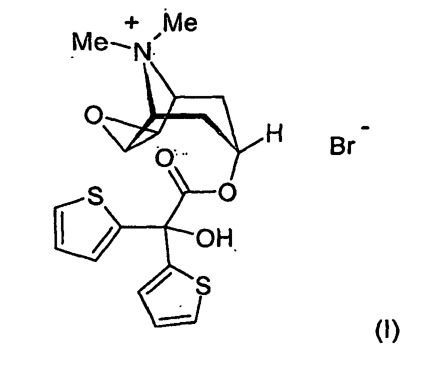

Die Verbindung besitzt wertvolle pharmakologische Eigenschaften und ist unter dem Namen Tiotropiumbromid bekannt. Tiotropium und seine Salze stellen ein hochwirksame Anticholinergika dar und können deshalb bei der Therapie von Asthma oder COPD (chronic obstructive pulmonary disease = chronisch obstruktive Lungenerkrankung) einen therapeutischen Nutzen entfalten. Pharmakologisch ebenfalls interessant ist das Monohydrat des Tiotropiumbromids.
Beide Verbindungen sind bevorzugter Gegenstand der vorliegenden Erfindung.

Die vorliegende Erfindung beschäftigt sich mit inhalativ applizierbaren flüssigen Wirkstoffformulierungen dieser Verbindungen, wobei die erfindungsgemäßen flüssigen Formulierungen hohen Qualitätsstandards genügen müssen. Um eine optimale Wirkstoffverteilung der Wirksubstanzen in der Lunge zu erhalten; bietet sich die Applikation einer flüssigen, auf Treibgase verzichtenden, Formulierung mittels dafür geeignet Inhalatoren an. Besonders geeignet sind solche Inhalatoren, die eine kleine Menge einer flüssigen Formulierung in der therapeutisch notwendigen Dosierung binnen weniger Sekunden in ein therapeutisch-inhalativ geeignetes Aerosol vernebeln können. Im Rahmen der vorliegenden Erfindung sind solche Vernebler bevorzugt, bei denen bereits eine Menge von weniger als 100 Mikroliter, bevorzugt weniger als 50 Mikroliter, ganz bevorzugt weniger als 20 Mikroliter Wirkstofflösung mit bevorzugt einem Hub zu einem Aerosol mit einer durchschnittlichen Teilchengröße von weniger als 20 Mikrometern, bevorzugt weniger als 10 Mikrometern, so vernebelt werden können, daß der inhalierbare Anteil des Aerosols bereits der therapeutisch wirksamen Menge entspricht.
Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 "Atomizing Device and Methods" als auch in der WO 97/12687, dort Figuren 6a und 6b und der dazugehörigen Beschreibung, ausführlich beschrieben. In einem solchen Vernebler wird eine Arzneimittellösung mittels hohen Drucks von bis zu 500 bar in ein lungengängiges Aerosol überführt und versprüht.
In solchen Inhalatoren werden die Lösungsformulierungen in einem Reservoir gelagert. Dabei ist es notwendig, daß die verwendeten Wirkstoffformulierungen eine ausreichende Lagerstabilität aufweisen und gleichzeitig so beschaffen sind, daß sie dem medizinischen Zweck entsprechend möglichst ohne weitere Manipulation, direkt appliziert werden können. Ferner dürfen sie keine Bestandteile aufweisen, die so mit dem Inhalator wechselwirken können, daß der Inhalator oder die pharmazeutische Qualität der Lösung, respektive des erzeugten Aerosols, Schaden nehmen könnte.

Zur Vernebelung der Lösung wird eine spezielle Düse verwendet, wie sie beispielsweise die WO 94/07607 oder die WO 99/16530 beschreibt.

Die WO 98/27959 offenbart Lösungsformulierungen für den oben beschriebenen Inhalator, die als Zusatz das Dinatriumsalz der Editinsäure (Natriumedetat) enthalten. Die Schrift favorisiert für wässrige Lösungsformulierungen, die mit Hilfe des eingangs beschriebenen Inhalators in inhalierbare Aerosole versprüht werden sollen, eine Mindestkonzentration an Natriumedetat von 50 mg/100 ml um die Inzidenz von Sprühanomalien zu verringern. Unter den offenbarten Beispielen findet sich eine Formulierung mit Tiotropiumbromid. Bei dieser Formulierung ist der Wirkstoff in Wasser gelöst. Der Anteil an Natriumedetat beträgt ebenfalls 50 mg / 100 ml.

Auch die WO 00/23037 offenbart Inhalationsformulierungen mit Tiotropiumbromid und EDTA.

Überraschend wurde jetzt gefunden, daß Lösungsformulierungen von Tiotropium-Salzen in Wasser oder einem Wasser-Ethanol-Gemisch, bei denen der Anteil des Zusatzstoffs Natriumedetat deutlich unterhalb von 50 mg/100 ml liegt, gegenüber der aus dem Stand der Technik bekannten Formulierung mit Tiotropiumbromid eine Reduktion der Streuung der ausgebrachten Masse aufweist. Zusätzlich ist die Sprayqualität sehr gut. Bevorzugt wird Wasser als Lösungsmittel eingesetzt.
Das daraus resultierende Aerosol weist für die inhalative Applikation sehr gute Eigenschaften auf.

Ein weiterer Vorteil der Formulierung besteht darin; daß durch den Verzicht bzw. die Reduktion des Zusatzstoffs Natriumedetat in der Wirkstoffformulierung der pH-Wert der Lösungsformulierung gesenkt werden kann. Niedrige pH-Werte sind der Langzeitstabilität der Formulierung förderlich.

Es ist daher eine Aufgabe der vorliegenden Erfindung, eine wässrige Wirkstoffformulierung mit einem pharmazeutisch akzeptablen Tiotropium-Salz zu schaffen, welche den hohen Standards genügt, die notwendig sind, um eine Lösung mittels der eingangs genannten Inhalatoren optimal vernebeln zu können. Die erfindungsgemäßen Wirkstoffformulierungen müssen dabei auch eine ausreichende pharmazeutische Qualität aufweisen, d.h. sie sollten über eine Lagerzeit von einigen Jahren, bevorzugt von mindestens einem Jahr, stärker bevorzugt von zwei Jahren pharmazeutisch stabil sein.

Eine weitere Aufgabe besteht darin, treibgasfreie Lösungsformulierungen mit Tiotropiumsalzen zu schaffen, die mittels eines Inhalators unter Druck vernebelt werden, wobei die im generierten Aerosol ausgebrachte Masse reproduzierbar innerhalb eines definierten Bereichs liegt.

Erfindungsgemäß können für die Formulierung alle pharmazeutisch akzeptablen Salze des Tiotropiums eingesetzten werden. Wird im Rahmen der vorliegenden Erfindung der Begriff Tiotropium-Salz verwendet, so ist dies als Bezugnahme auf Tiotropium zu verstehen. Eine Bezugnahme auf Tiotropium, welches das freie Ammoniumkation darstellt, entspricht erfindungsgemäß einer Bezugnahme auf Tiotropium in Form eines Salzes (Tiotropium-Salz), welches ein Anion als Gegenion enthält. Als im Rahmen der vorliegenden Erfindung einsetzbare Tiotropium-Salze sind bevorzugt Verbindungen zu verstehen, die neben Tiotropium als Gegenion (Anion) Chlorid, Bromid, lodid, Methansulfonat, para-Toluolsulfonat und/oder Methylsulfat enthalten.

Im Rahmen der vorliegenden Erfindung ist als Salz das Tiotropiumbromid bevorzugt. Bezugnahmen auf Tiotropiumbromid sind im Rahmen der vorliegenden Erfindung stets als Bezugnahmen auf alle möglichen amorphen und kristallinen Modifikationen des Tiotropiumbromids zu verstehen. Diese können beispielsweise in der kristallinen Struktur Lösemittelmoleküle mit einschließen. Von allen kristallinen Modifikationen des Tiotropiumbromids sind erfindungsgemäß diejenigen, die Wasser mit einschließen (Hydrate) bevorzugt. Besonders bevorzugt ist im Rahmen der vorliegenden Erfindung das Tiotropiumbromid-Monohydrat einsetzbar.
Die Formulierung enthält bevorzugt keinen weiteren Wirkstoff, der nicht Tiotropium oder ein pharmazeutisch verträgliches Salz davon ist.

In den erfindungsgemäßen Formulierungen liegen die Tiotropium-Salze in einem Lösungsmittel gelöst vor. Dabei kann das Lösungsmittel ausschließlich Wasser sein, oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Bevorzugtes Lösungsmittel ist Wasser ohne ethanolischen Zusatz.
Erfindungsgemäß enthält die Formulierung bevorzugt nur ein einziges Tiotropiumsalz. Allerdings kann die Formulierung auch ein Gemisch verschiedener Tiotropiumsalze und Solvate enthalten. Bevorzugt enthält die Formulierung keinen von Tiotropium im Sinn der obigen Definition verschiedenen Wirkstoff.

Die Konzentration des Tiotropium-Salzes bezogen auf den Anteil an Tiotropium in der fertigen Arzneimittelzubereitung ist abhängig von dem angestrebten therapeutischen Effekt. Für die Mehrzahl der auf Tiotropium ansprechenden Erkrankungen liegt die Konzentration an Tiotropium zwischen 0,0005 und 5 Gew. %, bevorzugt zwischen 0,001 und 3 Gew.%. Im Fall des Tiotropiumbromids bzw. Tiotropiumbromid-Monohydrats liegt die bevorzugte Menge bezogen auf Tiotropium bei 0,0005 bis 0,5 Gew.%, stärker bevorzugt bei 0,0005 bis 0,25 Gew.% und besonders bevorzugt bei 0,001 bis 0.1 Gew. %.

Der pH-Wert der erfindungsgemäßen Formulierung liegt zwischen 2,0 und 3,1 bevorzugt zwischen 2,5 und 3,1 und stärker bevorzugt zwischen 2,7 und 3,1 und besonders bevorzugt zwischen 2,7 und 3,1.

Der pH-Wert wird durch Zugabe von pharmakologisch verträglichen Säuren eingestellt.
Beispiele für diesbezüglich bevorzugte anorganische Säuren sind: Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit dem Wirkstoff ein Säureadditionssalz bilden.
Unter den organischen Säuren sind Ascorbinsäure, Fumarsäure und Zitronensäure bevorzugt, wobei Zitronensäure am stärksten bevorzugt ist. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe oder Antioxidantien besitzen, wie beispielsweise Zitronensäure oder Ascorbinsäure.
Als anorganische Säuren wird ausdrücklich Salzsäure genannt.

Gegebenenfalls können auch pharmakologisch verträgliche Basen zum genauen Austitrieren des pH-Wertes eingesetzt werden. Als Basen eignen sich beispielsweise Alkalihydroxide und Alkalicarbonate. Bevorzugtes Alkaliion ist Natrium. Werden solche Basen verwendet, ist darauf zu achten, daß auch die daraus resultierenden Salze, die dann in der fertigen Arzneimittelformulierung enthalten sind, mit der oben genannten Säure pharmakologisch akzeptabel ist.

Erfindungsgemäß kann in der vorliegenden Formulierung auf den Zusatz von Editinsäure (EDTA) oder einem der bekannten Salze davon, Natriumedetat, als Stabilisator oder Komplexbildner verzichtet werden.

Eine andere Ausführungsform beinhaltet Editinsäure und/oder die genannten Salze davon.

In einer solchen bevorzugten Ausführungsform liegt der Gehalt bezogen auf Natriumedetat unter 10 mg /100 ml. In diesem Fall findet sich ein bevorzugter Bereich zwischen 5 mg/100 ml und kleiner 10 mg/100 ml oder ein anderer zwischen größer 0 und 5 mg/100ml.

In einer anderen Ausführungsform beträgt der Gehalt an Natriumedetat 10 bis zu 25 mg/100 ml.

In einer bevorzugten Ausführungsform wird auf diesen Zusatz gänzlich verzichtet.

Analoges wie bereits für Natriumedetat ausgeführt, gilt auch für andere vergleichbare Zusatzstoffe, die komplexbildende Eigenschaften aufweisen und anstelle dessen verwendet werden können, wie beispielsweise Nitrilotriessigsäure und deren Salze.

Unter Komplexbildner werden im Rahmen der vorliegenden Erfindung bevorzugt Moleküle verstanden, die in der Lage sind Komplexbindungen einzugehen. Bevorzugt sollen durch diese Verbindungen Kationen, besonders bevorzugt metallische Kationen komplexiert werden.

Der erfindungsgemäßen Formulierung können neben Ethanol auch weitere Co-Solventien und/oder weitere Hilfsstoffe zugesetzt werden.

Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester, sofern sie nicht bereits das Lösungs- oder Suspensionsmittel sind.

Unter Hilfs- und Zusatzstoffen wird in diesem Zusammenhang jeder pharmakologisch verträgliche und therapeutisch sinnvolle Stoff verstanden, der kein Wirkstoff ist, aber zusammen mit dem Wirkstoff in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Sorbitantrioleat, Polyvinylpyrrolidon sonstige Stabilisatoren, Komplexbildner, Antioxidantien und/oder Konservierungsstoffe, die die Verwendungsdauer der fertigen Arzneimittelformulierung verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit pathogenen Keimen zu schützen. Als Konservierungsstoffe eignen sich die dem Stand der Technik bekannten, insbesondere Benzalkoniumchlorid oder Benzoesäure bzw. Benzoate wie Natriumbenzoat in der aus dem Stand der Technik bekannten Konzentration.

Bevorzugte Formulierungen enthalten außer dem Lösungsmittel Wasser und dem Tiotropiumsalz nur noch Benzalkoniumchlorid und Natriumedetat.
In einer anderen bevorzugten Ausführungsform wird auf Natriumedetat verzichtet.

Wie bereits mehrfach erwähnt, wird Tiotropiumbromid in der EP 418 716 A1 beschrieben, kristallines Tiotropiumbromid-Monohydrat kann mittels eines Herstellverfahrens, welches nachfolgend detaillierter beschrieben wird, erhalten werden.
Zur Herstellung des kristallinen Monohydrats gemäß der vorliegenden Erfindung ist es erforderlich, Tiotropiumbromid, welches beispielsweise nach der in der EP 418 716 A1 offenbarten Herstellungsvorschrift erhalten worden ist, in Wasser aufzunehmen, zu Erwärmen, eine Reinigung mit Aktivkohle durchzuführen und nach Abtrennen der Aktivkohle unter langsamem Abkühlen das Tiotropiumbromid-Monohydrat langsam zu kristallisieren.
Bevorzugt wird wie nachfolgend beschrieben vorgegangen.
In einem geeignet dimensionierten Reaktionsgefäß wird das Lösemittel mit Tiotropiumbromid, welches beispielsweise nach der in der EP 418 716 A1 offenbarten Herstellungsvorschrift erhalten worden ist, gemischt. Pro Mol eingesetztes Tiotropiumbromid werden 0,4 bis 1,5 kg, bevorzugt 0,6 bis 1 kg, besonders bevorzugt ca. 0,8 kg Wasser als Lösemittel verwendet.
Die erhaltene Mischung wird unter Rühren erwärmt, vorzugsweise auf mehr als 50°C, besonders bevorzugt auf mehr als 60°C. Die maximal wählbare Temperatur bestimmt sich durch den Siedepunkt des verwendeten Lösemittels Wasser. Vorzugsweise wird die Mischung auf einen Bereich von 80-90°C erhitzt.
In diese Lösung wird Aktivkohle, trocken oder wasserfeucht, eingebracht. Bevorzugt werden pro Mol eingesetztes Tiotropiumbromid 10 bis 50 g, besonders bevorzugt 15 bis 35 g, höchst bevorzugt etwa 25 g Aktivkohle eingesetzt. Gegebenenfalls wird die Aktivkohle vor Einbringen in die Tiotropiumbromid-haltige Lösung in Wasser aufgeschlämmt. Pro Mol eingesetztes Tiotropiumbromid werden zum Aufschlämmen der Aktivkohle 70 bis 200 g, bevorzugt 100 bis 160 g, besonders bevorzugt ca. 135 g Wasser verwendet. Wird die Aktivkohle vor Einbringen in die Tiotropiumbromid-haltige Lösung zuvor in Wasser aufgeschlämmt, empfiehlt es sich, mit der gleichen Menge Wasser nachzuspülen.
Bei konstanter Temperatur wird nach erfolgter Aktivkohlezugabe zwischen 5 bis 60 Minuten, bevorzugt-zwischen 10 und 30 Minuten, besonders bevorzugt etwa 15 Minuten weitergerührt und die erhaltene Mischung filtriert, um die Aktivkohle zu entfernen. Der Filter wird anschließend mit Wasser nachgespült. Hierfür werden pro Mol eingesetztes Tiotropiumbromid 140 bis 400 g, bevorzugt 200 bis 320 g, höchst bevorzugt ca. 270 g Wasser verwendet.
Das Filtrat wird anschließend langsam abgekühlt, vorzugsweise auf eine Temperatur von 20-25°C. Die Abkühlung wird vorzugsweise mit einer Abkühlrate von 1 bis 10°C pro 10 bis 30 Minuten, bevorzugt von 2 bis 8°C pro 10 bis 30 Minuten, besonders bevorzugt von 3 bis 5°C pro 10 bis 20 Minuten, höchst bevorzugt von 3 bis 5°C pro ca. 20 Minuten durchgeführt. Gegebenenfalls kann sich nach dem Abkühlen auf 20 bis 25°C eine weitere Abkühlung auf unter 20°C, besonders bevorzugt auf 10 bis 15°C anschließen.
Nach erfolgter Abkühlung wird zwischen 20 Minuten und 3 Stunden, vorzugsweise zwischen 40 Minuten und 2 Stunden, besonders bevorzugt etwa eine Stunde zur Vervollständigung der Kristallisation nachgerührt.
Die entstandenen Kristalle werden abschließend durch Filtrieren oder Absaugen des Lösemittels isoliert. Sollte es erforderlich sein, die erhaltenen Kristalle einem weiteren Waschschritt zu unterwerfen, empfiehlt es sich als Waschlösemittel Wasser oder Aceton zu verwenden. Pro Mol eingesetztes Tiotropiumbromid können zum Waschen der erhaltenen Tiotropiumbromid-monohydrat-Kristalle 0,1 bis 1,0 L, bevorzugt 0,2 bis 0,5 L, besonders bevorzugt etwa 0,3 L Lösemittel Verwendung finden. Gegebenenfalls kann der Waschschritt wiederholt durchgeführt werden.
Das erhaltene Produkt wird im Vakuum oder mittels erwärmter Umluft bis zum Erreichen eines Wassergehalts von 2,5 - 4,0 % getrocknet.

Ein Aspekt der vorliegenden Erfindung betrifft daher auch Lösungsformulierungen der oben beschriebenen Art, bei denen kristallines Tiotropiumbromid-Monohydrat eingesetzt wird, welches gemäß vorstehend beschriebener Vorgehensweise erhältlich ist.

Die erfindungsgemäßen Arzneimittelformulierungen mit Tiotropium-Salzen werden bevorzugt in einem Inhalator der vorstehend beschriebenen Art verwendet, um daraus die erfindungsgemäßen treibgasfreien Aerosole herzustellen.

Wie eingangs geschildert wird eine weiterentwickelte Ausführungsform des bevorzugten Inhalators in der WO 97/12687 und deren Figuren 6 offenbart. Dieser Vernebler (Respimat®) kann vorteilhaft zur Erzeugung der erfindungsgemäßen inhalierbaren Aerosole mit einem Tiotropium-Salz als Wirkstoff eingesetzt werden. Aufgrund seiner zylinderähnlichen Form und einer handlichen Größe von weniger als 9 bis 15 cm in der Länge und 2 bis 4 cm in der Breite kann dieses Device jederzeit vom Patienten mitgeführt werden. Der Vernebler versprüht ein definiertes Volumen der Arzneimittelformulierung unter Anwendung hoher Drücke durch kleine Düsen, so daß inhalierbare Aerosole entstehen.

Im wesentlichen besteht der bevorzugte Zerstäuber aus einem Gehäuseoberteil, einem Pumpengehäuse, einer Düse, einem Sperrspannwerk, einem Federgehäuse, einer Feder und einem Vorratsbehälter, gekennzeichnet durch
- ein Pumpengehäuse, das im Gehäuseoberteil befestigt ist, und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- ein Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist.

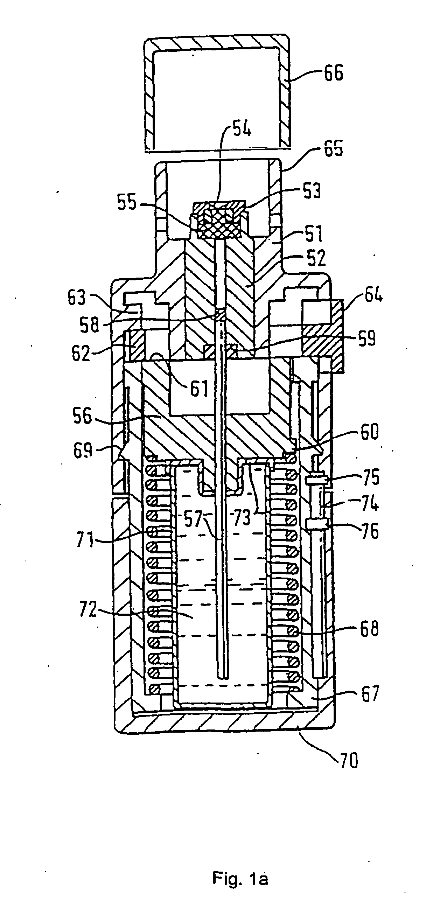

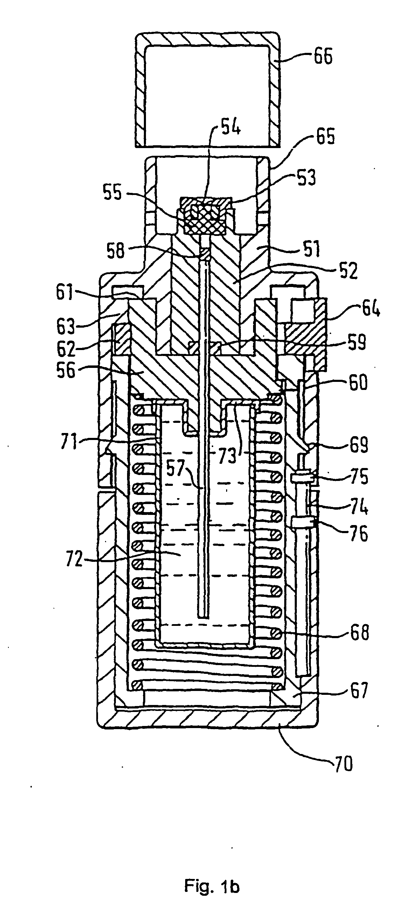

Der Hohlkolben mit Ventilkörper entspricht einer in der WO 97/12687 offenbarten Vorrichtungen. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 MPa (etwa 50 bis 600 bar), bevorzugt 10 bis 60 MPa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 10 bis 20 Mikroliter, ganz besonders bevorzugt ist ein Volumen von 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO-94/07607 offenbart; auf diese Schrift wird hiermit inhaltlich Bezug genommen, insbesondere auf Figur 1 und deren Beschreibung.
Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlaßseite mit der Düsenauslaßseite verbinden. Auf der Düsenauslaßseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Länge bei 7 bis 9 Mikrometern beträgt.
Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslaßseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 30 bis 70 Mikrometer. Am stärksten bevorzugt sind 50 Mikrometer.
Die Strahlrichtungen treffen sich dementsprechend in der Umgebung der Düsenöffnungen.

Die flüssige Arzneimittelzubereitung trifft wie bereits erwähnt mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchengrößen des Aerosols liegen bei bis zu 20 Mikrometern, bevorzugt 3 bis 10 Mikrometern.

Das Sperrspannwerk enthält eine Feder, bevorzugt eine zylindrische schraubenförmige Druckfeder, als Speicher für die mechanische Energie. Die Feder wirkt auf den Abtriebsflansch als Sprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sprerrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseobereil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Weitere konstruktive Details sind in den PCT-Anmeldungen WO 97/12683 und WO 97/20590 offenbart.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

In den Figuren 1a/b, die identisch sind mit den Figuren 6 a/b der WO 97/12687, ist der Vernebler (Respimat®) beschrieben, mit dem die erfindungsgemäßen wäßrigen Aerosolzubereitungen vorteilhaft inhaliert werden können.

Figur 1a zeigt einen Längsschnitt durch den Zerstäuber bei gespannter Feder, Figur 1 b zeigt einen Längsschnitt durch den Zerstäuber bei entspannter Feder.

Das Gehäuseoberteil (51) enthält das Pumpengehäuse (52), an dessen Ende der Halter (53) für die Zerstäuberdüse angebracht ist. In dem Halter befindet sich der Düsenkörper (54) und ein Filter (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseobereil endet im Mundstück (65) und ist mit der aufsteckbaren Schutzkappe (66) verschlossen.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht. An dem Ende der Spindel, das dem Gehäuseoberteil zugewandt ist, befindet das Antriebsritzel (75). Auf der Spindel sitzt der Reiter (76).

Der oben beschriebene Vernebler ist geeignet, die erfindungsgemäßen Aerosolzubereitungen zu einem für die Inhalation geeignetem Aerosol zu vernebeln.

Wird die erfindungsgemäße Formulierung mittels der vorstehend beschriebenen Technik (Respimat®) vernebelt, sollte die ausgebrachte Masse bei wenigstens 97%, bevorzugt wenigstens 98% aller Betätigungen des Inhalators (Hube) einer definierten Menge mit einem Toleranzbereichs von maximal 25%, bevorzugt 20% dieser Menge entsprechen. Bevorzugt werden pro Hub zwischen 5 und 30 mg Formulierung als definierte Masse ausgebracht, besonders bevorzugt zwischen 5 und 20 mg.

Die erfindungsgemäße Formulierung kann jedoch auch mittels anderer als der vorstehend beschriebenen Inhalatoren, beispielsweise Jet-Stream-Inhalatoren, vernebelt werden.

### Beispiele

### I. Synthesebeispiel für Tiotropiumbromid-Monohydrat

In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 Min. bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei 25°C über 2 Stunden im Stickstoffstrom getrocknet.
Ausbeute : 13,4 kg Tiotropiumbromid-Monohydrat (86 % d. Th.)

### II. Formulierungsbeispiele

100 g Arzneimittelzubereitung enthalten:

| Beispiel | Menge an Tiotropiumbromid mit einem Anteil bezogen auf Tiotropium: | Menge an Tiotrpoimbromid-Monohydrat mit einem Anteil bezogen auf Tiotropium: | Menge an Benzalkonium-chlorid | Menge an Natrium-edetat | pH-Wert, eingestellt mit HCl (1N) |
|---|---|---|---|---|---|
| 1 | 0,099 g | --- | 10 mg | 25 mg | 3,0 |
| 2 | 0,006 g | --- | 10 mg | 25 mg | 3,0 |
| 3 | 0,099 g | --- | 10 mg | 10 mg | 3,0 |
| 4 | 0,006 g | --- | 10 mg | 10 mg | 3,0 |
| 5 | --- | 0,099 g | 10 mg | 25 mg | 3,0 |
| 6 | --- | 0,006 g | 10 mg | 25 mg | 3,0 |
| 7 | --- | 0,099 g | 10 mg | 10 mg | 3,0 |
| 8 | --- | 0,006 g | 10 mg | 10 mg | 3,0 |

Der restliche Bestandteil ist Wasser.

## Patentansprüche

1. Treibgasfreie Arzneimittelzubereitung zur inhalativen Applikation bestehend aus
• einem Tiotropium-Salz als Wirkstoff, in einer Konzentration bezogen auf Tiotropium zwischen 0,0005 und 5 Gew. %,
• nur Wasser oder einem Wasser/Ethanol-Gemisch als Lösungsmittel für den Wirkstoff,
• Säure zum Einstellen eines pH-Werts zwischen 2,0 und 3,1,
• einem pharmakologisch verträglichen Konservierungsmittel,
• optional Editinsäure oder einem Editinsäuresalz in einer Menge von größer 0 bis zu 25 mg/100 ml,
• optional einem pharmakologisch akzeptablen Komplexbildner und/oder Stabilisator und/oder einem pharmakologisch akzeptablen Co-Solvens und/oder andere pharmakologisch akzeptablen Hilfs- und Zusatzstoffen neben dem Konservierungsmittel.

2. Arzneimittelzubereitung nach Anspruch 1, **dadurch gekennzeichnet, daß** das Tiotropiumsalz Chlorid, Bromid, lodid, Methansulfonat, para-Toluolsulfonat oder Methylsulfat ist.

3. Arzneimittelzubereitung nach Anspruch 1, **dadurch gekennzeichnet, daß** der Wirkstoff Tiotropiumbromid ist.

4. Arzneimittelzubereitung nach Anspruch 1, **dadurch gekennzeichnet, daß** der Wirkstoff Tiotropiumbromid-Monohydrat ist.

5. Arzneimittelzubereitung nach einem der Ansprüche 1 bis 4, **dadurch gekennzeichnet, daß** das Lösungsmittel nur Wasser ist.

6. Arzneimittelzubereitung nach einem der Ansprüche 1 bis 4, **dadurch gekennzeichnet, daß** das Lösungsmittel ein Wasser-Ethanol-Gemisch ist mit bevorzugt bis zu 70 Vol.% Ethanol, besonders bevorzugt bis zu 60 Vol.% Ethanol und ganz besonders bevorzugt bis zu 30 Vol.% Ethanol.

7. Arzneimittelzubereitung nach einem der Ansprüche 1 bis 6, **dadurch gekennzeichnet, daß** kein Komplexbildner enthalten ist.

8. Arzneimittelzubereitung nach einem der Ansprüche 1 bis 7, **dadurch gekennzeichnet, daß** kein Stabilisator enthalten ist.

9. Arzneimittelzubereitung nach einem der Ansprüche 1 bis 6, **dadurch gekennzeichnet, daß** das Editinsäuresalz in einer Menge von 5 bis kleiner 10 mg /100 ml enthalten ist.

10. Arzneimittelzubereitung nach einem der Ansprüche 1 bis 6, **dadurch gekennzeichnet, daß** das Editinsäuresalz in einer Menge von 10 bis 25 mg /100 ml enthalten ist.

11. Arzneimittelzubereitung nach Ansprüche 1 bis 6 oder 9 bis 10, **dadurch gekennzeichnet, daß** das Editinsäuresalz Natriumedetat ist.

12. Arzneimittelzubereitung nach einem der Ansprüche 1 bis 11, **dadurch gekennzeichnet, daß** der pH-Wert zwischen 2,5 und 3,1, bevorzugt zwischen 2,7 und 3,1 und ganz besonders bevorzugt zwischen 2,7 und 3,0 liegt.

13. Arzneimittelzubereitung nach einem der Ansprüche 1 bis 12, **dadurch gekennzeichnet, daß** die Konzentration bezogen auf Tiotropium zwischen 0,001 und 3 Gew.%, bevorzugt 0,0005 bis 0,5 Gew.%, besonders bevorzugt 0,0005 bis 0,25 Gew.% und besonders bevorzugt bei 0,001 bis 0,1 Gew. % beträgt.

14. Arzneimittelzubereitung nach einem der Ansprüche 1 bis 13, **dadurch gekennzeichnet, daß** die Zubereitung Benzalkoniumchlorid als Konservierungsmittel enthält.

15. Arzneimittelzubereitung nach einem der Ansprüche 1 bis 14, **dadurch gekennzeichnet, daß** pharmakologisch akzeptablen Hilfs- und Zusatzstoffe neben dem Konservierungsmittel verwendet werden.

16. Arzneimittelzubereitung nach Anspruch 15, **dadurch gekennzeichnet, daß** die Zubereitung als Hilfsstoff ein Antioxidans enthält.

17. Arzneimittelzubereitung nach einem der Ansprüche 1 bis 16, **dadurch gekennzeichnet, daß** keine Co-Solventien und/oder pharmakologisch akzeptablen Hilfs- und Zusatzstoffe außer dem Konservierungsmittel verwendet werden.

18. Verwendung einer Arzneimittelzubereitung nach einem der Ansprüche 1 bis 17 zur Herstellung eines Arzneimittels zur Behandlung von Asthma und/oder COPD.

19. Verfahren zur Herstellung einer Arzneimittelzubereitung nach einem der Ansprüche 1 bis 17 durch Mischen der einzelnen Komponenten.

## Claims

1. Propellant-free pharmaceutical preparation for administration by inhalation comprising
• a tiotropium salt as active substance, in a concentration based on tiotropium of between 0.0005 and 5 % by weight,
• water on its own or a water/ethanol mixture as solvent for the active substance,
• acid for achieving a pH between 2.0 and 3.1,
• a pharmacologically acceptable preservative,
• optionally editic acid or an editic acid salt in an amount of greater than 0 up to 25 mg/100 ml,
• optionally a pharmacologically acceptable complexing agent and/or stabiliser and/or a pharmacologically acceptable cosolvent and/or other pharmacologically acceptable adjuvants and additives in addition to the preservative.

2. Pharmaceutical preparation according to claim 1, **characterised in that** the tiotropium salt is chloride, bromide, iodide, methanesulphonate, *para*toluenesulphonate or methylsulphate.

3. Pharmaceutical preparation according to claim 1, **characterised in that** the active substance is tiotropium bromide.

4. Pharmaceutical preparation according to claim 1, **characterised in that** the active substance is tiotropium bromide monohydrate.

5. Pharmaceutical preparation according to one of claims 1 to 4, **characterised in that** the solvent is water on its own.

6. Pharmaceutical preparation according to one of claims 1 to 4, **characterised in that** the solvent is a water-ethanol mixture with preferably up to 70 vol.% of ethanol, more preferably up to 60 vol. % of ethanol and most preferably up to 30 vol. % of ethanol.

7. Pharmaceutical preparation according to one of claims 1 to 6, **characterised in that** it does not contain a complexing agent.

8. Pharmaceutical preparation according to one of claims 1 to 7, **characterised in that** it does not contain a stabiliser.

9. Pharmaceutical preparation according to one of claims 1 to 6, **characterised in that** the editic acid salt is present in an amount from 5 to less than 10 mg /100 ml.

10. Pharmaceutical preparation according to one of claims 1 to 6, **characterised in that** the editic acid salt is present in an amount from 10 to 25 mg /100 ml.

11. Pharmaceutical preparation according to claims 1 to 6 or 9 to 10, **characterised in that** the editic acid salt is sodium edetate.

12. Pharmaceutical preparation according to one of claims 1 to 11, **characterised in that** the pH is between 2.5 and 3.1, preferably between 2.7 and 3.1, and most preferably between 2.7 and 3.0.

13. Pharmaceutical preparation according to any one of claims 1 to 12, **characterised in that** the concentration based on tiotropium is between 0.001 and 3 % by weight, preferably 0.0005 to 0.5% by weight, particularly preferably 0.0005 to 0.25% by weight, and particularly preferably 0.001 to 0.1% by weight.

14. Pharmaceutical preparation according to any one of claims 1 to 13, **characterised in that** the preparation contains benzalkonium chloride as preservative.

15. Pharmaceutical preparation according to one of claims 1 to 14, **characterised in that** pharmacologically acceptable adjuvants and additives are used in addition to the preservative.

16. Pharmaceutical preparation according to claim 15, **characterised in that** the preparation contains an antioxidant as adjuvant.

17. Pharmaceutical preparation according to one of claims 1 to 16, **characterised in that** no cosolvents and/or pharmacologically acceptable adjuvants and additives are used apart from the preservative.

18. Use of a pharmaceutical preparation according to one of claims 1 to 17 for preparing a medicament for the treatment of asthma and/or COPD.

19. Process for preparing a pharmaceutical preparation according to one of claims 1 to 17 by mixing the individual components.

## Revendications

1. Préparation médicamenteuse sans gaz propulseur pour l'application par inhalation consistant en
• un sel de tiotropium comme principe actif, en une concentration rapportée au tiotropium entre 0,0005 et 5 % en masse,
• de l'eau seulement ou un mélange eau/éthanol comme solvant pour le principe actif,
• un acide pour établir un pH entre 2,0 et 3,1,
• un conservateur pharmacologiquement acceptable,
• éventuellement de l'acide édétique ou un sel de l'acide édétique en une quantité de plus de 0 à 25 mg/100ml,
• éventuellement un complexant et/ou stabilisant pharmacologiquement acceptable et/ou un cosolvant pharmacologiquement acceptable et/ou d'autres adjuvants et additifs pharmacologiquement acceptables outre le conservateur.

2. Préparation médicamenteuse selon la revendication 1 **caractérisée en ce que** le sel de tiotropium est le chlorure, le bromure, l'iodure, le méthanesulfonate, le para-toluènesulfonate ou le méthylsulfate.

3. Préparation médicamenteuse selon la revendication 1 **caractérisée en ce que** le principe actif est le bromure de tiotropium.

4. Préparation médicamenteuse selon la revendication 1 **caractérisée en ce que** le principe actif est le bromure de tiotropium monohydraté.

5. Préparation médicamenteuse selon l'une des revendications 1 à 4 **caractérisée en ce que** le solvant est l'eau seulement.

6. Préparation médicamenteuse selon l'une des revendications 1 à 4 **caractérisée en ce que** le solvant est un mélange eau-éthanol avec de préférence jusqu'à 70 vol. % d'éthanol, de manière particulièrement préférée jusqu'à 60 vol. % d'éthanol et de manière tout particulièrement préférée jusqu'à 30 vol. % d'éthanol.

7. Préparation médicamenteuse selon l'une des revendications 1 à 6 **caractérisée en ce qu'**aucun complexant n'est contenu.

8. Préparation médicamenteuse selon l'une des revendications 1 à 7 **caractérisée en ce qu'**aucun stabilisant n'est contenu.

9. Préparation médicamenteuse selon l'une des revendications 1 à 6 **caractérisée en ce que** le sel de l'acide édétique est contenu en une quantité de 5 à moins de 10 mg/100 ml.

10. Préparation médicamenteuse selon l'une des revendications 1 à 6 **caractérisée en ce que** le sel de l'acide édétique est contenu en une quantité de 10 à 25 mg/100 ml.

11. Préparation médicamenteuse selon les revendications 1 à 6 ou 9 à 10 **caractérisée en ce que** le sel de l'acide édétique est l'édétate de sodium.

12. Préparation médicamenteuse selon l'une des revendications 1 à 11 **caractérisée en ce que** le pH est situé entre 2,5 et 3,1, de préférence entre 2,7 et 3,1 et de manière tout particulièrement préférée entre 2,7 et 3,0.

13. Préparation médicamenteuse selon l'une des revendications 1 à 12 **caractérisée en ce que** la concentration rapportée au tiotropium est entre 0,001 et 3 % en masse, de préférence 0,0005 à 0,5 % en masse, de manière particulièrement préférée 0,0005 à 0,25 % en masse et de manière particulièrement préférée 0,001 à 0,1 % en masse.

14. Préparation médicamenteuse selon l'une des revendications 1 à 13 **caractérisée en ce que** la préparation contient du chlorure de benzalkonium comme conservateur.

15. Préparation médicamenteuse selon l'une des revendications 1 à 14 **caractérisée en ce que** des adjuvants et additifs pharmacologiquement acceptables sont utilisés outre le conservateur.

16. Préparation médicamenteuse selon la revendication 15 **caractérisée en ce que** la préparation contient un antioxydant comme adjuvant.

17. Préparation médicamenteuse selon l'une des revendications 1 à 16 **caractérisée en ce qu'**aucun cosolvant et/ou adjuvant et additif pharmacologiquement acceptable n'est utilisé hormis le conservateur.

18. Utilisation d'une préparation médicamenteuse selon l'une des revendications 1 à 17 pour la production d'un médicament pour le traitement de l'asthme et/ou de la COPD.

19. Procédé de production d'une préparation médicamenteuse selon l'une des revendications 1 à 17 par mélange des composants individuels.
